**Europäisches Patentamt**

**European Patent Office**

**Office européen des brevets**

(19)

(11) Numéro de publication : **0 151 074**
**B1**

(12)

# FASCICULE DE BREVET EUROPÉEN

(45) Date de publication du fascicule du brevet :
21.10.87

(51) Int. Cl.⁴ : **C 07 C 79/04**, C 07 B 43/02

(21) Numéro de dépôt : **85400118.7**

(22) Date de dépôt : **25.01.85**

---

(54) **Procédé de fabrication de nitrométhane et installation.**

---

(30) Priorité : **27.01.84 FR 8401248**

(43) Date de publication de la demande :
**07.08.85 Bulletin 85/32**

(45) Mention de la délivrance du brevet :
**21.10.87 Bulletin 87/43**

(84) Etats contractants désignés :
**AT BE CH DE FR GB IT LI LU NL SE**

(56) Documents cités :
**EP-A- 0 085 326**
**FR-A- 1 581 000**
**FR-A- 2 158 708**
**US-A- 2 161 475**
**US-A- 2 291 345**
**US-A- 2 597 698**

(73) Titulaire : **SOCIETE CHIMIQUE DE LA GRANDE PAROISSE, AZOTE ET PRODUITS CHIMIQUES**
**8, rue Cognacq-Jay**
**F-75007 Paris (FR)**

(72) Inventeur : **Jacquinot, Bernard**
**59, rue Léon Bathiat**
**F-59500 Douai (FR)**
Inventeur : **Quibel, Jacques**
**40, rue de la Muette**
**F-78600 Maisons-Laffitte (FR)**
Inventeur : **Mari, Roger**
**8, avenue de Brabois**
**F-54600 Villers-lès-Nancy (FR)**

(74) Mandataire : **Bouton Neuvy, Liliane et al**
**L'Air liquide, Société Anonyme pour L'Etude et L'Exploitation des Procédés Georges Claude 75, Quai d'Orsay**
**F-75321 Paris Cedex 07 (FR)**

Jouve, 18, rue St-Denis, 75001 Paris, France

## Description

La présente invention concerne la fabrication de nitrométhane ou de mélanges de nitroparaffines à forte teneur en nitrométhane.

Divers procédés ont été proposés pour fabriquer des nitroparaffines à partir d'hydrocarbures aliphatiques, en particulier propane, éthane et de mélanges. Toutefois, ces techniques ne permettent pas de résoudre au mieux le problème actuel de la production industrielle de nitroparaffines. Pour atteindre un niveau satisfaisant de rentabilité, il est nécessaire de produire un spectre de nitroparaffines adapté à la demande du marché, tout en utilisant des matières premières peu onéreuses et disponibles en grosses quantités. Il est d'autre part, indispensable de limiter la consommation spécifique globale en hydrocarbures, qui tient compte tant de la consommation dans le réacteur que de la perte en hydrocarbures dans l'opération de récupération de ceux-ci.

Même, si la mise en œuvre d'éthane ou de mélanges éthane-propane selon les brevets. Français 2 421 867, 2 442 828 et 2 453 846 donnent des moyens d'accroissement de la teneur en nitrométhane dans les nitroparaffines produites, il est apparu que la nitration directe du méthane devrait constituer une solution plus avantageuse que les précédentes.

Cependant, il est bien connu que le méthane est beaucoup plus malaisé à nitrer que les hydrocarbures plus lourds, en raison de sa plus grande stabilité — donc de la difficulté plus importante à rompre une liaison carbone-hydrogène — et de la plus grande fragilité de la molécule de nitrométhane qui risque d'être décomposée dans le réacteur après sa formation. Les précédents travaux relatés dans les brevets américains 2 161 475, 2 164 774, 2 418 241, 2 512 587 et 4 329 523 enseignent des conditions de nitration du méthane. Et le brevet US 2 291 345 concerne la nitration du méthane par l'acide nitrique en phase vapeur, avec des temps de contact très brefs de l'ordre de 0,005 à 1 seconde, et des températures relativement élevées entre 375 et 550 °C, en présence d'un gaz inerte, l'azote qui accroît le rendement de nitration. Cependant les taux de conversion du méthane en nitrométhane sont toujours restés faibles ; la conversion maximale, obtenue en introduisant l'acide nitrique en plusieurs points, n'a pas dépassé 7 %.

Du fait d'une récupération délicate du méthane n'ayant pas réagi dans le réacteur, des rapports d'hydrocarbures/agent nitrant élevés (minimum 8 en rapport molaire) et des faibles conversions du méthane en nitrométhane, les méthodes de l'art antérieur n'ont pas conduit à une consommation spécifique globale en méthane suffisamment faible pour envisager une exploitation industrielle.

Le brevet US 2 597 698 est relatif à la nitration des hydrocarbures aliphatiques, en particulier du propane avec de l'acide nitrique en présence d'un halogène. Cet halogène peut être sous la forme d'hydrures, d'halogénures d'alkyle et d'acyle. Et, il est fait remarquer que ce procédé est particulièrement avantageux pour les paraffines de $C_2$ à $C_8$.

On a recherché, un procédé de nitration du méthane, ou d'un mélange d'hydrocarbures riche en méthane tel les gaz naturels, ou les gaz de raffinerie par un agent de nitration, avec un taux de conversion important et une quantité résiduaire en méthane suffisamment faible pour qu'une récupération ne soit pas nécessaire.

Il a été trouvé que pour obtenir les rendements et conversions attendus, les rapports quantitatifs des divers réactifs, le temps de contact réactionnel, la température et la pression de nitration sont choisis et contrôlés de manière telle que l'ensemble du mélange réactionnel : hydrocarbure, agent nitrant et autres constituants éventuels, soit mis en œuvre en phase gazeuse homogène.

L'agent nitrant est l'acide nitrique et le peroxyde d'azote utilisés seuls ou en mélange. L'acide nitrique peut avoir une concentration comprise entre 30 et 100 %, et plus avantageusement de 65 à 100 %. On peut utiliser l'acide nitrique industriel de concentration maximale 60-65 %.

Le choix du rapport molaire méthane-agent nitrant influe sur les résultats, avantageusement celui-ci doit être compris entre 0,1 et 5, de préférence entre 0,2 et 2.

Le temps de contact réactionnel est fonction de la température, de la pression et du rapport méthane/agent nitrant, avantageusement entre 0,1 et 120 secondes, et préférentiellement entre 5 et 30 secondes.

La pression de nitration est maintenue entre 1 et 35 bars absolus et préférentiellement entre 2 et 10 bars avec l'acide nitrique, et 5 à 30 bars absolus avec le peroxyde d'azote.

Les températures de réaction sont comprises entre 270 °C et 600 °C, et de préférence entre 300 et 480 °C. Elles sont rigoureusement contrôlées par les moyens appropriés en vue d'assurer un transfert thermique.

Pour la mise en œuvre du procédé, les réactants sont préchauffés à température contrôlée au plus égale à la température de réaction ; l'agent nitrant étant préchauffé séparément du méthane ou du mélange d'hydrocarbures riches en méthane ; le mélange réactionnel étant réalisé à une température au plus égale à la température de réaction en un point le plus proche de la zone réactionnelle, et de manière la plus homogène possible.

De plus, il a été constaté que le taux de conversion du méthane en nitrométhane pouvait être amélioré par l'addition d'agent actif au cours de la nitration. L'agent actif participant à la nitration est introduit à un débit tel que le rapport molaire agent actif/agent nitrant n'excède pas 3 ; ce rapport étant ajusté, en fonction des critères économiques, à sa valeur optimale.

Les halogènes, notamment chlore et brome et leurs dérivés halogénés tels les hydracides comme l'acide chlorhydrique ; les halogénures organiques tels les chlorures et bromures d'alkyle, mono ou polyhalogénés ou les halogénures d'alcène mono ou polyhalogénés susceptibles d'être vaporisés dans les conditions de la réaction constituent des agents actifs de choix, assimilables à des catalyseurs homogènes. Tout halogénure organique aliphatique ou arylique saturé ou insaturé convient à la mise en œuvre du procédé.

Si l'acide chlorhydrique, les composés chlorés et le chlore ont des comportements voisins, et de ce fait une influence comparable, les dérivés bromés, iodés et fluorés, selon leur stabilité, ont une action parfois moins déterminante. On peut citer en outre le chlorure de méthylène, le trichlorométhane, l'iodure de méthyle, le chlorure d'éthyle, le bromure d'éthyle, le chlorure de propyle et d'isopropyle, le fluorure d'isoamyle, le chlorure de benzyle, le dichloro et tétrachloroéthylène, mono, di, trichloréthane et tétrachlorure de carbone.

L'action de l'agent actif sur le rendement de conversion est sensible, intéressante et avantageuse quand l'agent actif est introduit dans un rapport molaire agent actif/méthane au plus égal à 3.

L'objet de la demande concerne donc un procédé de préparation de nitrométhane par nitration en phase gazeuse homogène de méthane ou de mélanges d'hydrocarbures riches en méthane, l'agent nitrant étant l'acide nitrique, le peroxyde d'azote ou leurs mélanges, en présence de constituant inerte vis-à-vis de la réaction et des produits réactés, caractérisé en ce que le rapport molaire méthane/agent nitrant est compris entre 0,2 et 2 ; le temps de contact réactionnel est compris entre 1 et 30 secondes ; la pression réactionnelle est comprise entre 2 et 10 bars quand l'agent nitrant est l'acide nitrique et entre 5 et 30 bars dans le cas du peroxyde d'azote ; la température réactionnelle étant comprise entre 300 et 480 °C ; et en ce que la réaction de nitration est conduite en présence d'un agent actif ou promoteur introduit dans un rapport molaire agent actif/méthane au plus égal à 3 ; l'agent actif étant un halogène, un hydracide, un dérivé organique halogéné, tel un halogénure d'alkyle, d'aryle, saturé ou insaturé, susceptible d'être vaporisé ou mis en solution dans un solvant organique ou aqueux.

Suivant sa nature chimique, l'agent actif est introduit dans le milieu réactionnel, en mélange avec le réactant vis-à-vis duquel il a un comportement neutre. Selon les réactions éventuelles entre l'agent actif et l'un des réactifs, qu'il convient d'éviter, ledit agent sera introduit soit avec l'agent nitrant soit avec l'hydrocarbure.

L'agent actif halogéné doit être vaporisable avec l'un des réactants, ou bien susceptible d'être mis en solution dans un solvant organique ou aqueux.

L'utilisation de l'agent actif en solution dans un solvant approprié conduit à des conversions au moins égales à celles qu'on pourrait obtenir avec l'agent actif employé pur. Ceci est particulièrement intéressant lors de l'emploi d'acide nitrique industriel (concentration maximale 60-65 %) comme agent nitrant.

D'autres composés, tels les porteurs de groupements NO ou $NO_2$, les aldéhydes, les cétones ou certains alcools peuvent être utilisés soit individuellement soit en association avec les composés chlorés précités. Les nitroparaffines, tels les nitropropanes contribuent à l'amélioration de la conversion.

En outre, dans certaines conditions la réaction de nitration peut être facilitée par l'addition d'un ou plusieurs constituants inertes vis-à-vis de la réaction et des produits réactés. L'inerte peut être constitué d'un gaz, d'un mélange gazeux ou d'un liquide volatil. L'utilisation d'acide nitrique de dilution choisie permet d'introduire l'inerte sous forme d'eau de dilution et de diminuer le coût de l'agent nitrant. Le recyclage d'une partie de la fraction non condensable des effluents du réacteur — après les épurations adéquates — permet également l'emploi au titre d'inerte des oxydes de carbone formés au cours de la réaction.

Le constituant inerte peut être choisi parmi des gaz tels l'azote, l'oxyde de carbone, le dioxyde de carbone, l'hydrogène ou un mélange de ces gaz, parmi des liquides vaporisables tels des vapeurs de composés organiques ou la vapeur d'eau provenant de la dilution de l'acide nitrique, ou parmi un mélange de ces gaz et de ces vapeurs.

Le procédé objet de l'invention permet d'envisager une exploitation industrielle répondant aux exigences économiques.

Le réacteur est le siège de la nitration, et tous les paramètres de la réaction : température, pression, rapports méthane/agent nitrant, débit d'agent actif, teneur en gaz inertes, composition du mélange employé le cas échéant comme agent actif, sont contrôlés avec précision ; les possibilités d'échange thermique au sein du réacteur étant suffisantes pour évacuer correctement la chaleur dégagée.

Le gaz riche en méthane contenant les inertes, ainsi que l'agent nitrant, sont préchauffés avant leur mise en réaction. L'addition de l'agent actif se fait soit dans l'agent nitrant dans le cas de l'acide chlorhydrique, soit dans le gaz riche en méthane pour un composé organique halogéné ou mélange.

On a constaté l'intérêt de la récupération de l'énergie thermique des effluents de nitration, au cours du refroidissement de ceux-ci, en vue de la condensation des produits liquides provenant de la réaction.

A partir du mélange gazeux effluent, exempt des produits liquides condensés, l'agent nitrant est récupéré par absorption des oxydes d'azote, tandis que le méthane n'ayant pas réagi et les inertes fabriqués au cours de la réaction, essentiellement les oxydes de carbone CO et $CO_2$ peuvent être pour une partie recyclés vers la réaction de nitration après recompression et complémentation par un appoint

3

**0 151 074**

en gaz riche en méthane, et pour l'autre partie évacués en vue de leur utilisation comme combustible soit dans l'unité elle-même, soit dans une unité voisine consommatrice d'énergie thermique.

L'effluent gazeux utilisé, comme combustible est soutiré à un débit régulé de manière à maintenir constante la teneur des inertes dans le mélange réactionnel.

La phase liquide issue de la réaction est dénitrée par traitement avec un gaz oxygéné en vue de l'oxydation des oxydes d'azote, de la récupération et du recyclage de l'agent nitrant, après concentration.

La fraction d'agent nitrant récupérée à partir de la phase gazeuse effluente après condensation, et la fraction récupérée à partir de la phase liquide effluente sont réunies, complétées par un appoint d'agent nitrant et recyclées vers la nitration.

Le mélange de nitroparaffines à haute teneur en nitrométhane est récupéré à partir de la phase liquide dénitrée, puis traité, et l'agent actif séparé sous forme d'hydracide peut être recyclé vers la réaction de nitration après addition dans l'agent nitrant.

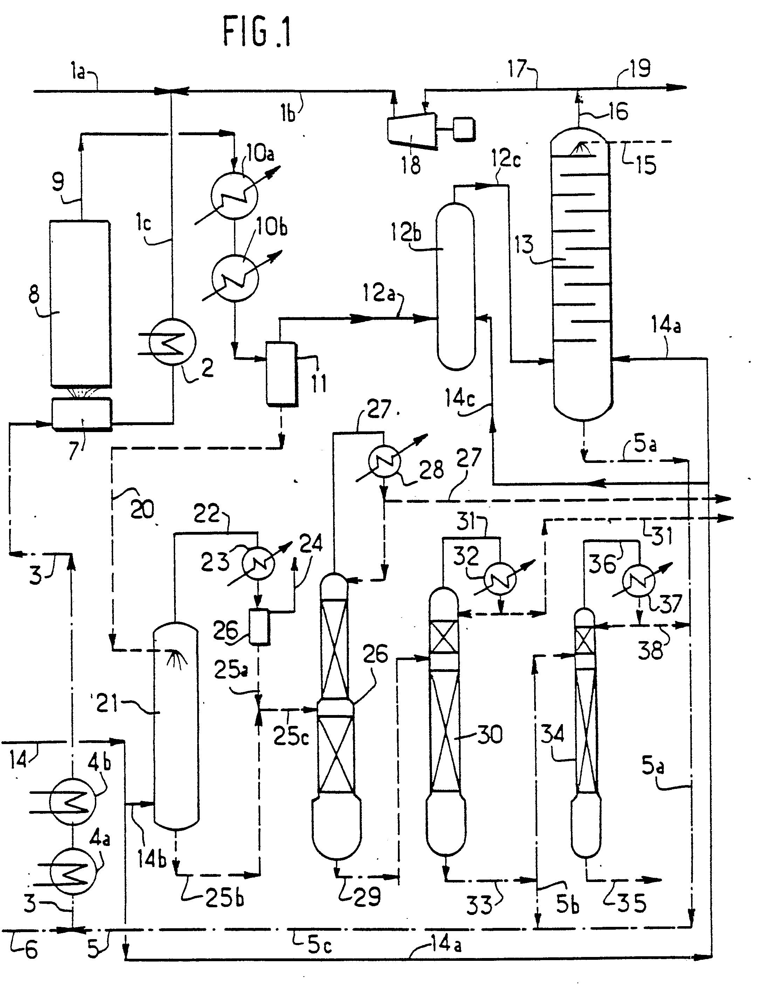

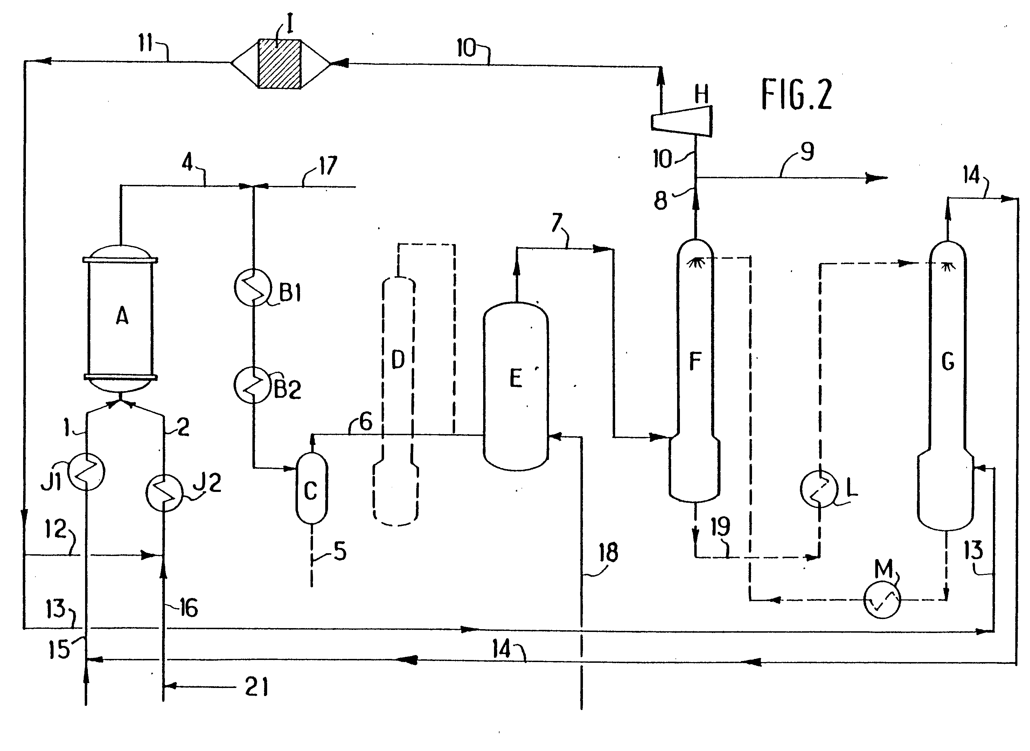

Une unité industrielle de fabrication de nitrométhane fonctionnant avec l'acide nitrique suivant le procédé décrit est représentée sur la figure I du dessin annexé. Dans le cas illustré on considère l'acide nitrique comme agent nitrant et l'acide chlorhydrique comme agent actif; les inertes essentiellement les oxydes de carbone sont fabriqués au cours de la nitration. L'installation fonctionne avec recyclage du méthane n'ayant pas réagi, des agents nitrant et actif et des inertes.

Le gaz riche en méthane (1c) contenant une quantité contrôlée d'inertes est préchauffé dans l'échangeur-réchauffeur (2). De son côté l'acide nitrique (3) de concentration requise est vaporisé puis surchauffé dans les échangeurs (4a) et (4b). Ce courant d'acide nitrique est constitué par les deux fractions d'acide nitrique récupérées et réunies en (6) et un appoint de $NO_3H$ (7).

Les deux courants de réactifs (1) et (3) méthane + inertes et acide nitrique + acide chlorhydrique sont mélangés en phase gazeuse homogène dans le mélangeur (7) avant d'être introduits dans le réacteur de nitration (8).

Les effluents (9) du réacteur sont refroidis dans les échangeurs de chaleur (10a) et (10b) de façon à assurer efficacement la condensation des produits liquides. L'échangeur (10a) peut être avantageusement constitué par une chaudière de récupération, destinée à récupérer une partie de l'énergie thermique des effluents.

La phase gazeuse effluente est ensuite séparée de la phase liquide effluente dans la zone de séparation constituée par le séparateur (11).

La phase gazeuse effluente (12a) est envoyée dans la zone d'absorption des oxydes d'azote, constituée par la colonne d'absorption (13), qui fonctionne selon les principes connus d'absorption des $NO_x$ mis en application dans les installations d'acide nitrique. Les oxydes d'azote contenus dans le mélange gazeux (12a) sont transformés en présence d'un gaz oxygéné, air ou oxygène (14-14a) en un acide nitrique (5a) de concentration voulue récupéré à la base de la colonne (13), tandis que le débit d'eau requis, pour obtenir la concentration fixée, est admis (15) en tête de ladite colonne.

Pour obtenir en pied de la colonne (13) un acide de concentration suffisamment élevée, il est avantageux de procéder à une oxydation préalable du mélange gazeux avant son introduction dans la colonne d'absorption (13). Cette oxydation des oxydes d'azote inférieurs par un gaz oxygéné (14a) (air-oxygène ou air suroxygéné) peut s'effectuer dans une tour d'oxydation (12b) du type de celles qui sont employées dans les installations d'acide nitrique.

Les gaz contenant le méthane n'ayant pas réagi, les inertes introduits en (1c) avec le mélange riche en méthane, ainsi que les inertes fabriqués au cours du processus de nitration, sont récupérés à partir du haut de la colonne d'absorption des oxydes d'azote en (16).

Une partie (17) de ce mélange gazeux est recomprimée dans le compresseur (18) pour être renvoyée par l'intermédiaire de la ligne (1b) vers la zone de nitration après avoir reçu l'appoint (1a) en gaz riche en méthane. L'autre partie (19) du mélange gazeux est évacuée à un débit déterminé de manière à maintenir constante la teneur des inertes dans le mélange (1c) envoyé à la nitration.

La phase liquide issue du séparateur (11) est envoyée par l'intermédiaire de la conduite (20) au sommet d'une zone d'oxydation des oxydes d'azote dissous en acide nitrique, constituée par la colonne (21), à l'intérieur de laquelle les liquides sont en contact à contre-courant avec le gaz oxygéné (14b). Une condensation efficace des vapeurs résiduaires d'oxydation (22) dans l'échangeur (23) permet de limiter l'entraînement du nitrométhane, les gaz résiduaires sont évacués en (24) et la phase liquide (25a) séparée dans le séparateur (26).

La phase liquide exempte d'oxydes d'azote $NO_x$ est recueillie par la ligne (25b) à la base de la colonne (21). Les deux fractions de la phase liquide riche en nitroparaffines sont réunies dans la ligne (25c).

Cette phase liquide (25c) ainsi dénitrée est conduite sur la colonne de distillation (26), où le nitrométhane ou le mélange de nitroparaffines, à haute teneur en nitrométhane, est récupéré en tête (27) sous forme d'azéotrope avec l'eau, à la pression atmosphérique, après refroidissement dans le refroidisseur (28), une partie de la fraction condensée peut être retournée en tête de la colonne (26).

L'acide chlorhydrique contenu dans la phase liquide (25c) est recueilli en pied de colonne dans le liquide (29) avec le mélange eau-acide nitrique ; grâce à un réglage approprié et contrôlé du fonctionnement de la colonne de séparation des nitroparaffines.

Si on utilise un chlorure organique comme agent actif, celui-ci se retrouve en partie sous forme

4

d'acide chlorhydrique en sortie du réacteur. Cet acide est recueilli en pied de la colonne dans le liquide (29) avec le mélange eau/acide nitrique, grâce à un réglage approprié de la colonne de séparation des nitroparaffines. La partie non transformée des chlorures organiques est récupérée soit en tête de colonne soit en pied selon la température d'ébullition. Ce chlorure séparé peut être recyclé.

Le liquide (29) soutiré en pied de la colonne de distillation (26) est soumis dans la colonne de distillation (30) à une distillation sous pression atmosphérique pour éliminer l'eau formée au cours de la réaction en tête de la colonne (31). La vapeur d'eau après refroidissement dans le refroidisseur (32) est évacuée, une fraction pouvant être recyclée en tête de colonne (30).

L'acide nitrique qui a subi une reconcentration est recueilli en pied de la colonne (30) à la concentration de 65 % en masse par la conduite (33).

Selon la composition de l'hydrocarbure riche en méthane utilisé, la réaction conduit à la formation d'une certaine quantité de produits lourds qu'il convient d'éliminer. Dans ce but, tout ou partie de l'acide nitrique reconcentré (33) est distillé dans la colonne (34). Les produits lourds sont recueillis en pied de colonne (35).

L'acide nitrique (5a) issu de la colonne d'absorption des oxydes d'azote (13), l'acide nitrique (33) issu de la colonne de concentration (30) et l'acide (38) issu de la colonne (34) sont mélangés, et après addition de l'appoint (6) en acide nitrique, sont recyclés au réacteur.

Afin d'obtenir un acide à recycler à une concentration suffisante et dans le but d'économiser une partie de l'énergie dissipée pour le chauffage des bouilleurs des colonnes (30) et (34), il est avantageux de prélever une partie de l'acide nitrique dilué (29) et de l'introduire au niveau approprié de la colonne d'absorption (13) tout en réduisant le débit d'eau (15) en tête de colonne.

On a procédé à des études en dynamique et en statique de l'influence des différents paramètres influant sur le degré de conversion du méthane en nitrométhane.

Le tableau I consigne les résultats de l'étude en dynamique et le tableau II permet de comparer en statique l'influence de différents composés halogénés sur la conversion du méthane.

Tableau I

Nitration du méthane en dynamique

| | | | | | | | | | |
|---|---|---|---|---|---|---|---|---|---|
| Volume du Réacteur ($cm^3$) | 106 | | | | | 250 | | | |
| Température °C | 390 | | | | | 370 | | | |
| Débit $CH_4$ (Nl/h) | 2.52 | | | | | 1.7 | 3.1 | 4.5 | 2.4 |
| Débit du mélange $HNO_3$/HCl ($cm^3$/h) | 9.5 | | | | | 13.1 | 11.8 | 8.5 | 8.8 |
| % vol. de solution aqueuse de HCl (à 37% masse) dans le mélange $HNO_3$ solution d'HCl | 0 | 1 | 2 | 5 | 10 | 5 | 5 | 5 | 5 |
| ($CH_4$/$HNO_3$) mol. | 0.50 | 0.50 | 0.51 | 0.52 | 0.55 | 0.26 | 0.52 | 1.04 | 0.54 |
| (HCl/$HNO_3$) mol. % | 0 | 0.5% | 1.% | 2.7% | 5.5% | 2.7% | 2.7% | 2.7% | 2.7% |
| Temps de séjour (secondes) | 20.8 | 20.5 | 19.5 | 19.3 | 18 | 40.4 | 37.8 | 40.1 | 50.2 |
| Pression (bar absolu) | 1 | 1 | 1 | 1 | 1 | 1 | 1 | 1 | 1 |
| % CONVERSION DE $CH_4$ EN NITRO METHANE | 2.1 | 7.4 | 14.1 | 15.6 | 18.4 | 21.5 | 16.4 | 6. | 18.7 |

(La désignation .10 signifie 0,10 et ainsi pour chaque quantité précédée d'un point) (ceci dans les tableaux I et II).

Ce tableau I met en évidence l'influence du rapport CH₄/HNO₃.

0 151 074

Tableau II

Expériences conduites dans des réacteurs statiques
Nitrations du méthane en présence de différents agents actifs
Agent nitrant = $HNO_3$ ou $NO_2$

| Volume du réacteur (cm³) | 700 | | | | 950 | 700 | 950 | 700 | | | 950 | | |
|---|---|---|---|---|---|---|---|---|---|---|---|---|---|
| Pression Initiale (mm. Hg) ** | 660 | 700 | 720 | 700 | 700 | 640 | 770 | 700 | 720 | 750 | 670 | 670 | 660 |
| TEMPERATURE (°C) | 400°C | | | | | | | | | | | | |
| Temps de Séjour (mn) | 5 mn | | | | | | | | | | | | |
| $CH_4$ introduit cm³ N.T.P. | 120 | 100 | 100 | 120 | 120 | 120 | 120 | 120 | 120 | 120 | 120 | 120 | 120 |
| $NO_2$ " cm³ liq | | 0.24 | 0.2 | | | 0.35 | 0.18 | 0.18 | | | | | |
| $HNO_3$ 100 % " " " | 0.24 | 0.3 | | 0.4 | 0.2 | | | 0.4 | 0.4 | 0.4 | 0.4 | 0.4 | 0.4 |
| AGENT ACTIF : Nature | — | — | — | — | HCl | HCl | HCl | HCl | HCl | HCl | $CH_2Cl_2$ | $CH_2Cl_2$ | $CH_2Cl_2$ |
| $CH_4$/Agent Nitrant (mol.) | 0.94 | 0.63 | 0.60 | 0.85 | 0.55 | 1.08 | 0.48 | 0.94 | 0.94 | 0.56 | 0.56 | 0.56 | 0.56 |
| Agent Actif/Agent Nitrant (% mol) | — | — | — | — | 2.5 | 2.5 | 2.2 | 2.1 | 4.2 | 5.1 | 2.9 | 4.1 | 1.6 |
| Conversion du Méthane en Nitrométhane (%) | 3.1 | 5.7 | 5.1 | 3.6 | 26. | 6.8 | 26.9 | 16.1 | 17.2 | 25.4 | 23.4 | 25.6 | 22.2 |

| Volume du réacteur (cm³) | 950 | | | 700 | | | 700 | |
|---|---|---|---|---|---|---|---|---|
| Pression Initiale (mm Hg) ** | 600 | 600 | 550 | 560 | 600 | 570 | 600 | 600 |
| TEMPERATURE (°C) | 360 | | | 380 | | | 380 | |
| Temps de Séjour (mn) | 5 mn | | | 5 mn | | | 5 mn | |
| $CH_4$ introduit cm³ NTP | 100 | 100 | 50 | 50 | | | 50 | |
| $HNO_3$ " cm³ liq. | 0.4 | 0.4 | 0.3 | 0.3 | | | 0.3 | |
| AGENT ACTIF : Nature | $Cl_2$ | $Br_2$ | | $CH_2Cl_2$ | | | CCl2=CCl2 | CHCl=CHCl |
| solvant utilisé : Nature | — | — | | Méthanol | | | Méthanol | Méthanol |
| vol. agent actif dans la sol. | 100% | 100 % | | 100% | 10% | 40% | 20% | 20% |
| volume solution introduit (cm³) | 4cm³ gaz | 0.02 | 0.02 | 0.02 | 0.04 | 0.02 | 0.04 | 0.04 |
| $CH_4$/$HNO_3$ (mol.) | 0.47 | 0.47 | 0.31 | 0.31 | | | 0.31 | 0.31 |
| Agent actif/Agent nitrant (mol) | 1.9 | 4.1 | 5.4 | 4.3 | 0.9 | 1.7 | 1.1 | 1.4 |
| Conversion du Méthane en Nitrométhane (%) | 22.6 | 5.5 | 5.9 | 26. | 20.5 | 28.2 | 30.4 | 28.8 |

** Calculée selon les quantités de réactifs introduits et la température du réacteur.

6

Le tableau III consigne des résultats d'étude de la nitration du méthane en dynamique par l'acide nitrique sous pression, en présence de divers agents actifs ou promoteurs.

Tableau III

| Agent nitrant | $HNO_3$ (conc. de 65 à 90 %) | | | | | |
|---|---|---|---|---|---|---|
| Température °C | 375 | 365 | 380 | 390 | 390 | 382 |
| Stoechiométrie | 0.26 | 0.30 | 0.24 | 0.22 | 0.28 | 0.18 |
| Pression (bar.abs) | 4 | 4 | 4 | 5 | 7 | 7 |
| Concentration % | 34 | 30,5 | 41,4 | 42,1 | 40,6 | 40,9 |
| Catalyse % | 2,9 | 4,9 | 1,4 | 1,01 | 1,24 | 0,7 |
| Temps de séjour en secondes | 11,4 | 11,1 | 12,2 | 11,7 | 9,9 | 9,7 |
| Nature du promoteur | $C_3H_7Cl$ | $C_3H_7Cl$ | $C_3H_7Cl$ | $C_2H_4Cl_2$ | $C_2H_4Cl_2$ | $C_2H_4Cl_2$ |
| Rendement en $NC_1$ | 10,8% | 8,95% | 8,15% | 12,7% | 16,1% | 15,8% |

Par stœchiométrie on désigne le rapport molaire hydrocarbures/agent nitrant à l'entrée du réacteur ; le terme concentration désigne la concentration des réactants exprimée en % molaire = [débit hydrocarbure + débit $HNO_3$/débit total gaz] × 100 ; le terme catalyse (exprimée en %) traduit la quantité de promoteur employé : Catalyse = Promoteur en moles/(hydrocarbures + $NO_2$) moles × 100 ; et le rendement en % : désigne le pourcentage de méthane transformé en nitrométhane = $NC_1$ formé (moles)/Méthane introduit (moles) × 100.

On a conduit une série d'essais de nitration en dynamique du méthane avec le peroxyde d'azote, en utilisant un réacteur de 386 cm³ de volume intérieur constitué par un tube de diamètre 8/10 mm et de 10 m de long. Les réactants : hydrocarbure et solution du promoteur d'une part, $NO_2$ et azote d'autre part, sont préchauffés à 135 °C et mélangés à l'entrée du réacteur.

Le promoteur étant le chlorure d'isopropyle, on a procédé à l'étude de l'influence de la catalyse, dont les résultats sont consignés dans le tableau IV.

Tableau IV

Influence de la Catalyse

| Agent nitrant $NO_2$ – Promoteur chlorure d'isopropyle | | | |
|---|---|---|---|
| Température (°C) | 344 | 360 | 375 |
| Stoechiométrie | 0,88 | | |
| Pression bar. | 9,7 | | |
| Concentration % | 80 | | |
| Catalyse % | 1,43 | 0,89 | 0,69 |
| temps de séjour (s) | 8,7 | 8,4 | 8,3 |
| Rendement en $NC_1$ % | 6,84 | 7,4 | 8,1 |

On a pu ainsi observer qu'une augmentation de la température compense avantageusement une baisse de la catalyse.

L'influence de la température a été étudiée dans le cadre de la nitration de $CH_4$ par $NO_2$ en présence de chlorure d'isopropyle.

## 0 151 074

Tableau V

Influence de la température

| Température °C | 360 | 375 | 400 |
|---|---|---|---|
| Stoechiométrie | 0,88 | 0,5 | 0,2 |
| Pression 1 bar abs. | 11,2 | | |
| Concentration % | 70 % | | |
| Catalyse % | 0,71 | 0,62 | 0,63 |
| Temps de séjour (s) | 8,4 | 8,4 | 8 |
| Rendement en $NC_1$ % | 5,4 | 10,2 | 26,7 |

Les résultats consignés dans ce tableau montrent que la diminution de la stœchiométrie de 0,88 à 0,2 associée à une augmentation de la température de 360 à 400 °C se traduit par une amélioration du rendement.

L'influence de la pression a été étudiée dans la nitration du méthane avec le peroxyde d'azote en présence de chlorure d'isopropyle.

Tableau VI

Influence de la pression

| Température °C | 387 | 386 | 393 |
|---|---|---|---|
| Stoechiométrie | 0,3 | | |
| Pression en bar.abs | 11 | 15,6 | 24,7 |
| Concentration % | 70 | 50 | 30 |
| Catalyse % | 0,67 | 0,62 | 0,62 |
| Temps de séjour (s) | 8 | | |
| Rendement en $NC_1$ | 16,6 | 19,1 | 20,4 |

Il apparaît que l'augmentation de pression, associée à une diminution de la concentration conduit à une plus grande augmentation de la sélectivité.

Ensuite on a procédé à une étude de l'influence de la nature du promoteur dans le cadre de la nitration du méthane par le peroxyde d'azote.

Tableau VII

Influence de la nature du promoteur

| Nature du promoteur | Chlorure d'iso-propyle | 1-2 dichloro éthane | | Tétrachlorure de carbone | |
|---|---|---|---|---|---|
| Température °C | 388 | 396 | 397 | 398 | 398 |
| Pression (bar) | 19,8 | 18,6 | 19 | 18,9 | 18,8 |
| Temps de séjour (s) | 8,3 | 7,8 | 7,8 | 8,1 | 8,1 |
| Concentration % | 40 | 40 | 40 | 40 | 40 |
| Stoechiométrie | 0,3 | 0,3 | 0,3 | 0,3 | 0,3 |
| Catalyse % | 0,53 | 0,20 | 0,27 | 0,12 | 0,10 |
| Rendement en $NC_1$ % | 17,6 | 19,2 | 23,3 | 20,7 | 18,5 |

8

# 0 151 074

On a pu observer que l'efficacité d'un promoteur chloré est due au nombre d'atomes de chlore qu'il contient.

Une unité industrielle de nitration du méthane par le peroxyde d'azote, éventuellement par le mélange acide nitrique et peroxyde d'azote est représentée sur la figure II du dessin annexé. Dans le cas illustré, l'installation fonctionne sous pression, et des essais conduisant à des résultats satisfaisants ont notamment été réalisés sous une pression de 19 bars. L'agent actif est un dérivé halogéné.

Le méthane d'appoint par le circuit d'alimentation (16) rejoint le gaz recyclé par le circuit de récupération (12) avant d'être préchauffé dans le réchauffeur ($J_2$). Dans un mélangeur approprié (non représenté) on réalise le mélange aussi homogène que possible du gaz (2) riche en méthane, auquel on a ajouté le dérivé chloré employé comme agent actif introduit par le circuit d'alimentation (21) et du gaz (1) riche en peroxyde d'azote. Cette opération s'effectue à proximité immédiate du réacteur (A). Ce réacteur peut être du type faisceau tubulaire en contact avec un fluide caloporteur de forte capacité d'échange thermique.

Les effluents (4) du réacteur subissent une trempe avec l'eau (17) et sont refroidis dans les refroidisseurs ($B_1$) et ($B_2$) de manière à condenser les constituants liquides qu'on sépare dans le séparateur (C) de la phase gazeuse (6). Le mélange liquide (5) est envoyé dans l'installation de lavage et de distillation où le nitrométhane sera récupéré ainsi que l'acide nitrique présent dans la phase liquide.

L'effluent gazeux (6) éventuellement lavé à l'eau dans le laveur (D) est conduit dans la tour d'oxydation (E) où, après addition du gaz (18) riche en oxygène (air, oxygène ou air suroxygéné), le monoxyde d'azote NO contenu est transformé en peroxyde d'azote $NO_2$ qui est absorbé, dans la colonne d'absorption (F) dans un solvant classique approprié, tel l'acide nitrique.

Le courant gazeux (8) est alors séparé en deux fractions. L'une, la ligne (9) constitue la purge de la boucle destinée à évacuer les inertes fabriqués ou introduits ; le débit de cette purge étant d'autant plus important que le gaz (18) contient plus d'inertes. Les gaz de purge (9) peuvent être utilisés comme combustibles (dans une chaudière par exemple), après qu'on ait récupéré le $NO_2$ encore contenu dans une colonne d'absorption.

L'autre fraction (10) du flux gazeux (8) est recomprimée dans le compresseur en (H) en vue de son retour au réacteur. De manière à éviter d'envoyer, avec les réactants, une quantité d'oxygène tel l'excès d'oxygène résultant de la transformation en E du NO en $NO_2$, qui serait préjudiciable à la nitration, on fait subir au gaz (10) une oxydation catalytique sélective dans l'oxydeur catalytique (I) pour brûler une partie du méthane contenu dans le gaz (10).

Le gaz (11) sortant de l'oxydeur (I) est alors séparé en deux fractions : la première (12) reçoit l'appoint de méthane ou de gaz riche en méthane : gaz naturel, gaz de raffinerie (16) et retourne directement au réacteur suivant (2).

L'autre fraction (13) est utilisée pour assurer la désorption du $NO_2$ dissous dans la solution (19) envoyée après chauffage dans le réchauffeur (L) dans la colonne de désorption (G). Le gaz riche en $NO_2$, sortant de la colonne (G) par le circuit de récupération (14), reçoit l'appoint de $NO_2$ par le circuit d'alimentation (15) avant de retourner au réacteur par la canalisation (1). La solution (20) appauvrie en $NO_2$ dissous, est refroidie dans le refroidisseur (M) avant d'être réutilisée dans l'absorbeur (F).

## Revendications

1. Procédé de préparation de nitrométhane par nitration en phase gazeuse homogène de méthane ou de mélanges d'hydrocarbures riches en méthane, l'agent nitrant étant l'acide nitrique, le peroxyde d'azote ou leurs mélanges, en présence de constituant inerte vis-à-vis de la réaction et des produits réactés, caractérisé en ce que le rapport molaire méthane/agent nitrant est compris entre 0,2 et 2 ; le temps de contact réactionnel est compris entre 1 et 30 secondes ; la pression réactionnelle est comprise entre 2 et 10 bars quand l'agent nitrant est l'acide nitrique et entre 5 et 30 bars dans le cas du peroxyde d'azote ; la température réactionnelle étant comprise entre 300 et 480 °C ; et en ce que la réaction de nitration est conduite en présence d'un agent actif ou promoteur introduit dans un rapport molaire agent actif/méthane au plus égal à 3 ; l'agent actif étant un halogène, un hydracide, un dérivé organique halogéné, tel un halogénure d'alkyle, d'aryle, saturé ou insaturé, susceptible d'être vaporisé ou mis en solution dans un solvant organique ou aqueux.

2. Procédé de préparation de nitrométhane selon la revendication 1, caractérisé en ce que l'agent actif est le chlore, l'acide chlorhydrique, le chlorure de méthylène, les dichloro et tétrachloroéthylène, le chlorure d'isopropyle, le 1,2-dichloroéthane ou le tétrachlorure de carbone.

3. Procédé de préparation de nitrométhane selon la revendication 1, caractérisé en ce que la réaction de nitration est conduite en présence d'une nitroparaffine en particulier un nitropropane.

4. Procédé de préparation de nitrométhane selon la revendication 1, caractérisé en ce que la réaction de nitration est conduite en présence d'un alcanol en particulier le méthanol.

5. Procédé de préparation de nitrométhane, selon une quelconque des revendications 1 à 4, caractérisé en ce que le méthane contenant les inertes et éventuellement l'agent actif, et l'agent nitrant contenant éventuellement l'agent actif, sont préchauffés indépendamment avant le mélange homogène en phase gazeuse de tous les constituants du mélange soumis à la nitration, en un point le plus proche

9

possible de la zone réactionnelle ; les effluents réactionnels sont séparés après refroidissement en une phase gazeuse et une phase liquide, à partir de ladite phase gazeuse, le méthane n'ayant pas réagi et les inertes fabriqués au cours de la nitration sont recyclés vers la nitration après recompression, et complémentation par un appoint en gaz riche en méthane ; en outre l'agent nitrant est récupéré par absorption des oxydes d'azote contenus dans la phase gazeuse ; la phase liquide issue de la réaction est dénitrée par traitement avec un gaz oxygéné en vue de la récupération de l'agent nitrant après concentration ; les fractions d'agent nitrant récupérées à partir des phases effluentes gazeuses et liquide sont réunies ; le mélange de nitroparaffines à haute teneur en nitrométhane étant récupéré à partir de la phase liquide dénitrée, pour être lavé et purifié.

6. Procédé de préparation de nitrométhane selon la revendication 5, quand l'agent nitrant est l'acide nitrique, caractérisé en ce que les fractions d'agent nitrant récupérées à partir des phases effluentes gazeuse et liquide sont réunies et recyclées vers la nitration, après complémentation par un appoint en agent nitrant.

7. Procédé de préparation de nitrométhane selon la revendication 5, quand l'agent actif est un hydracide tel l'acide chlorhydrique, caractérisé en ce que ledit agent actif est additionné dans l'agent nitrant et préchauffé en présence de celui-ci ; après la réaction de nitration, il est récupéré à partir de la phase liquide effluente dénitrée et recyclé.

8. Procédé de préparation de nitrométhane selon la revendication 5, quand l'agent actif est un composé organique halogéné, tel un chlorure organique, ledit agent est additionné dans le gaz riche en méthane, et préchauffé en présence de celui-ci ; et l'acide chlorhydrique issu de sa transformation au cours de la nitration est recyclé avec l'acide nitrique retourné au réacteur, tandis que le chlorure organique non transformé est séparé puis recyclé.

9. Procédé de préparation de nitrométhane selon la revendication 5, quand l'agent nitrant est le peroxyde d'azote, caractérisé en ce qu'une fraction de l'effluent gazeux issu de l'absorption du peroxyde d'azote est comprimée, soumise à une oxydation catalytique sélective, une fraction de cet effluent oxydé assurant la désorption du peroxyde d'azote dissous.

10. Installation de mise en œuvre industrielle du procédé de préparation du nitrométhane selon l'une quelconque des revendications 1 à 8, quand l'agent nitrant est l'acide nitrique et en présence d'un agent actif, caractérisée en ce qu'elle comprend le circuit d'alimentation (1c) en méthane et inertes, sur lequel est intercalé l'échangeur-réchauffeur (2), et le circuit d'alimentation (3) en agent nitrant et agent actif sur lequel sont intercalés les réchauffeurs (4a) et (4b), connectés au mélangeur (7) alimentant le réacteur de nitration (8), les échangeurs de chaleur (10a) et (10b) des effluents réactionnels, du séparateur (11) de la colonne d'oxydation des oxydes d'azote inférieurs (12b) de la colonne d'absorption des oxydes d'azote (13), du circuit de récupération (17) du méthane et des inertes, du compresseur (18) et de la ligne d'alimentation (1b) du méthane de recyclage et des inertes et de la ligne (1a) de l'appoint en méthane, la colonne de traitement de la phase liquide (21) par un gaz oxygéné ; la colonne de séparation des nitroparaffines (26), la colonne de concentration de l'acide nitrique (30) et la colonne d'élimination des produits lourds (34) ; et le circuit de récupération et de recyclage de l'agent nitrant (5a) (5b) (5c) et la ligne d'appoint en agent nitrant (6).

11. Installation de mise en œuvre industrielle du procédé de préparation du nitrométhane quand l'agent nitrant est le peroxyde d'azote, selon la figure II et les revendications 5 et 9, caractérisée en ce qu'elle comprend le circuit d'alimentation (16) en méthane d'appoint sur lequel sont connectés le circuit d'alimentation (21) en agent actif composé halogéné, hydracide ou dérivé halogéné, et le circuit de récupération (12) du gaz contenant du méthane, puis le circuit d'alimentation (15) en peroxyde d'azote d'appoint sur lequel est connecté le circuit de récupération (14) du gaz riche en $NO_2$, sur lesquels sont intercalés les réchauffeurs ($J_2$) et ($J_1$), un mélangeur des fluides (1) et (2) provenant des réchauffeurs, non représenté, à proximité du réacteur de nitration du type faisceau tubulaire (A), les refroidisseurs ($B_1$) et ($B_2$) des effluents (4), du séparateur (C), éventuellement du laveur (D), de la colonne d'oxydation (E) des oxydes d'azote inférieurs, de la colonne d'absorption du peroxyde d'azote (F), du compresseur (H) du flux gazeux retournant au réacteur de nitration par les circuits de récupération (11) et (12) après oxydation catalytique dans l'oxydeur (I), et la colonne de désorption (G) du peroxyde d'azote par une fraction du gaz sortant de l'oxydeur catalytique (I).

**Claims**

1. Process for the preparation of nitromethane by nitration of methane or mixtures of hydrocarbons rich in methane in homogeneous gaseous phase, the nitrating agent being nitric acid, nitrogen peroxide or mixtures thereof, in presence of a constituent inert against the reaction and the reaction products, characterized in that the molar ratio of methane/nitrating agent is between 0.2 and 2 ; the reaction contact time is between 1 and 30 sec, the reaction pressure is between 2 and 10 bar, if the nitrating agent is nitric acid and between 5 and 30 bar in the case of nitrogen peroxide ; the reaction temperature being between 300 and 480 ºC ; and that the nitration reaction is carried out in presence of an active agent or promotor introduced in a molar ratio of active agent/methane of at most 3 ; the active agent being a halogen, a hydrogen acid, an organic halogenated derivative, such as a saturated or unsaturated alkyl or aryl halogenide, able to be vapourized or solubilized in an organic or aqueous solvent.

2. Process for the preparation of nitromethane according to claim 1, characterized in that the active agent is chlorine, hydrogen chloride, methylen chloride, dichloro and tetrachloro ethylene, isopropyl chloride, 1,2-dichloro ethane or carbon tetrachloride.

3. Process for the preparation of nitromethane according to claim 1, characterized in that the nitration reaction is carried out in presence of a nitroparaffine, especially nitropropane.

4. Process for the preparation of nitromethane according to claim 1, characterized in that the nitration reaction is carried out in presence of an alkanol, especially methanol.

5. Process for the preparation of nitromethane according to one of the claims 1 to 4, characterized in that the methane containing the inerts and eventually the active agent and the nitrating agent containing eventually the active agent are preheated independently before the homogeneous mixture in gaseous phase of all of the constituents of the mixture is subjected to the nitration, at a closest possible point of the reaction zone, the reaction effluents are separated after cooling into a gaseous phase and a liquid phase, from the said gaseous phase the unreacted methane and the inerts prepared during the nitration are recycled to the nitration after recompression and supplemented by an addition of methane-riched gas, moreover, the nitrating agent is recovered by absorption of nitrogen oxides contained in the gaseous phase ; the liquid phase resulting from the reaction is denitrated by treatment with an oxygen containing gas for the recovery of the nitrating agent after concentration ; the fractions of nitrating agent recovered from the gaseous and liquid effluent phases are united, the mixture of nitroparaffins of high content of nitromethane being recovered from the denitrated liquid phase to be washed and purified.

6. Process for the preparation of nitromethane according to claim 5, if the nitrating agent is nitric acid, characterized in that the fractions of nitrating agent recovered from the gaseous and liquid effluent phases are united and recycled to the nitration after supplementing by an addition of nitrating agent.

7. Process for preparation of nitromethane according to claim 5, if the active agent is a hydrogen acid such as nitrogen chloride, characterized in that the said active agent is added in the nitrating agent and preheated in presence of the same ; after the reaction of nitration it its recovered from the denitrated liquid effluent phase and recycled.

8. Process for the preparation of nitromethane according to claim 5, if the active agent is a halogenated organic compound such as an organic chloride, characterized in that the said agent is added in the methane-rich gas and preheated in presence of the same and the hydrogen chloride resulting from the transformation during the nitration is recycled with the nitric acid returned to the reactor, while the organic chloride not transformed is separated and then recycled.

9. Process for the preparation of nitromethane according to claim 5, if the nitrating agent is nitrogen peroxide, characterized in that a gaseous fraction of the effluent resulting from the absorption of nitrogen peroxide is compressed and subjected to a selective catalytic oxidation, a fraction of said oxidized assuring the desorption of solved nitrogen peroxide.

10. Apparatus for industrially carrying out the process for preparation of nitromethane according to one of the claims 1 to 8, if the nitrating agent is nitric acid and in presence of an active agent, characterized in that it comprises the feed line (1c) for methane and inerts into which the exchanger-heater (2) is intercalated, and the feed line (3) for nitrating agent and active agent into which the heaters (45a) and (4b) are intercalated and which are connected with a mixer (7) feeding the reactor of nitration (8), the heat exchangers (10a) and (10b) for reaction effluents, a separator (11), the column of oxidation of the inferior nitrogen oxides (12b), the column of the absorption of nitrogen oxides (13), the recovery line (17) for methane and the inerts, a compressor (18) and a feed line (1b) for recycled methane and inerts and the line (1a) of the addition of methane, the column of treatment of the liquid phase (21) by an oxygen containing gas, the column of separation of nitroparaffins (26), the column of concentration of nitric acid (30) and the column of elimination of heavy products (34) and the line of recovery and recycling of the nitrating agent (5a), (5b), (5c) and the line of addition of nitrating agent (6).

11. Apparatus for industrially carrying out the process for the preparation of nitromethane, if the nitrating agent is nitrogen peroxide, according to Fig. II and the claims 5 and 9, characterized in that it comprises the feed line (16) for methane addition with which the feed line (21) for active agent, halogenated compound, hydrogen acid or halogenated derivative, and the recovery line (12) for methane containing gas are connected, then the feed line (15) for nitrogen peroxide addition with which the recovery line (14) for $NO_2$-rich gas is connected, into which the heaters $(J_2)$ and $(J_1)$ are intercalated, the mixture for fluids (1) and (2) comming from the heaters, not shown, in proximity of the nitration reactor of the tubular bundle type (C), eventually a washer (D), the column (E) for the oxidation of inferior nitrogen oxides, the column for absorption of nitrogen oxide (F), the compressor (H) for the gaseous stream returning to the nitration reactor by the recovery lines (11) and (12) of the catalytic oxidation in the oxidizer (I) and the column (G) for the desorption of nitrogen peroxide by the fraction of the gas leaving the catalytic oxidizer (I).

**Patentansprüche**

1. Verfahren zur Herstellung von Nitromethan durch Nitrierung von methanreichen Kohlenwasserstoffgemischen in homogener Gasphase, wobei das Nitriermittel Salpetersäure, Stickstoffperoxid oder deren Gemische ist, in Gegenwart eines gegenüber der Reaktion und den Reaktionsprodukten inerten Bestandteils, dadurch gekennzeichnet, daß das Molverhältnis von Methan/Nitriermittel zwischen 0,2 und 2 liegt, die Reaktionskontaktzeit zwischen 1 und 30 sec liegt, der Reaktionsdruck zwischen 2 und 10 bar, wenn das Nitriermittel Salpetersäure ist, und zwischen 5 und 30 bar im Falle, daß es Stickstoffperoxid ist, liegt und die Reaktionstemperatur zwischen 300 und 480 °C liegt, und daß man die Nitrierungsreaktion in Gegenwart eines aktiven Mittels oder Promotors durchführt, das bzw. der in einem Molverhältnis von aktivem Mittel zu Methan von höchstens 3 eingeführt wird, wobei das aktive Mittel ein Halogen, eine Wasserstoffsäure, ein halogeniertes organisches Derivat, wie ein gesättigtes oder ungesättigtes Alkyl- oder Arylhalogenid, die verdampfbar oder in einem organischen oder wäßrigen Lösungsmittel in Lösung bringbar sind, ist.

2. Verfahren zur Herstellung von Nitromethan nach Anspruch 1, dadurch gekennzeichnet, daß das aktive Mittel Chlor, Chlorwasserstoffsäure, Methylenchlorid, Dichlor- oder Tetrachlorethylen, Isopropylchlorid, 1,2-Dichlorethan oder Tetrachlorkohlenstoff ist.

3. Verfahren zur Herstellung von Nitromethan nach Anspruch 1, dadurch gekennzeichnet, daß die Nitrierungsreaktion in Gegenwart eines Nitroparaffins, besonders von Nitropropan, durchgeführt wird.

4. Verfahren zur Herstellung von Nitromethan nach Anspruch 1, dadurch gekennzeichnet, daß die Nitrierungsreaktion in Gegenwart eines Alkanols, besonders von Methanol, durchgeführt wird.

5. Verfahren zur Herstellung von Nitromethan nach einem der Ansprüche 1 bis 4, dadurch gekennzeichnet, daß das die Inertstoffe und gegebenenfalls aktives Mittel enthaltende Methan und das eventuell aktives Mittel enthaltende Nitriermittel unabhängig, bevor das homogene Gemisch in Gasphase aller Mischungsbestandteile der Nitrierung unterzogen wird, an einem Punkt der Reaktionszone so nahe wie möglich vorerhitzt werden, die Reaktionsausläufe nach Kühlung in eine Gasphase und eine flüssige Phase getrennt werden, von dieser Gasphase das unumgesetzte Methan und die im Verlaufe der Nitrierung gewonnenen Inertstoffe nach Wiederkomprimieren und Ergänzen durch einen Zusatz von methanreichem Gas zu der Nitrierung zurückgeführt werden, außerdem das Nitriermittel durch Absorption der in der Gasphase enthaltenen Stickstoffoxide wiedergewonnen wird ; die durch die Reaktion entstandene flüssige Phase durch Behandlung mit einem sauerstoffreichen Gas im Hinblick auf die Wiedergewinnung des Nitriermittels nach einer Konzentrierung denitriert wird ; die aus den gasförmigen und flüssigen Auslaufphasen wiedergewonnenen Nitriermittelfraktionen vereinigt werden und das Nitroparaffingemisch mit hohem Nitromethangehalt aus der denitrierten flüssigen Phase gewonnen wird, um gewaschen und gereinigt zu werden.

6. Verfahren zur Herstellung von Nitromethan nach Anspruch 5, wenn das Nitriermittel Salpetersäure ist, dadurch gekennzeichnet, daß die aus den gasförmigen und flüssigen Auslaufphasen wiedergewonnenen Nitriermittelfraktionen vereinigt und nach Ergänzung durch einen Nitriermittelzusatz zu der Nitrierung zurückgeführt werden.

7. Verfahren zur Herstellung von Nitromethan nach Anspruch 5, wenn das aktive Mittel eine Wasserstoffsäure, wie Chlorwasserstoffsäure, ist, dadurch gekennzeichnet, daß das aktive Mittel in dem Nitriermittel zugesetzt und in Gegenwart desselben vorerhitzt wird und daß es nach der Nitrierungsreaktion aus der denitrierten flüssigen Auslaufphase gewonnen und rückgeführt wird.

8. Verfahren zur Herstellung von Nitromethan nach Anspruch 5, wenn das aktive Mittel eine halogenierte organische Verbindung, wie ein organisches Chlorid, ist, dadurch gekennzeichnet, daß dieses Mittel in dem methanreichen Gas zugesetzt und in Gegenwart desselben vorerhitzt wird und daß die durch ihre Umwandlung im Verlaufe der Nitrierung gebildete Chlorwasserstoffsäure mit der zum Reaktor zurückgeführten Salpetersäure zurückgeführt wird, während das nicht umgewandelte organische Chlorid dann getrennt zurückgeführt wird.

9. Verfahren zur Herstellung von Nitromethan nach Anspruch 5, wenn das Nitriermittel Stickstoffperoxid ist, dadurch gekennzeichnet, daß eine bei der Absorption von Stickstoffperoxid gebildete gasförmige Auslauffraktion komprimiert und einer selektiven katalytischen Oxidation unterzogen wird, wobei eine Fraktion dieses oxidierten Auslaufs die Desorption von gelöstem Stickstoffperoxid gewährleistet.

10. Vorrichtung zur industriellen Durchführung des Verfahrens zur Herstellung von Nitromethan nach einem der Ansprüche 1 bis 8, wenn das Nitriermittel Salpetersäure ist, in Gegenwart eines aktiven Mittels, dadurch gekennzeichnet, daß sie die Einspeisleitung (1c) für Methan und Inertstoffe, in welche der Tauscher-Erhitzer (2) eingeschaltet ist, und die Einspeisleitung (3) für Nitriermittel und aktives Mittel, in welche die Erhitzer (4a) und (4b) eingeschaltet sind und die mit einem den Nitrierungsreaktor (8) speisenden Mischer (7) verbunden sind, die Wärmeaustauscher (10a) und (10b) für Reaktionsausläufe, eine Trenneinrichtung (11), die Kolonne für die Oxidation von niederen Stickstoffoxiden (12b), die Kolonne für die Absorption von Stickstoffoxiden (13), eine Wiedergewinnungsleitung (17) für Methan und Inertstoffe, einen Kompressor (18) und die Einspeisleitung (1b) für Rückführmethan und Inertstoffe und die Leitung (1a) für einen Methanzusatz, die Kolonne für die Behandlung der flüssigen Phase (21) mit einem sauerstoffhaltigen Gas, die Kolonne zur Trennung der Nitroparaffine (26), die Kolonne zur Konzentrierung der Salpetersäure (30) und die Kolonne zur Beseitigung der schweren Produkte (34) und die Leitung zur Gewinnung und Rückführung des Nitriermittels (5a), (5b), (5c) und die Leitung für den Zusatz von Nitriermittel (6) aufweist.

11. Vorrichtung zur industriellen Durchführung des Verfahrens zur Herstellung von Nitromethan, wenn das Nitriermittel Stickstoffperoxid ist, nach Fig. II und den Ansprüchen 5 und 9, dadurch gekennzeichnet, daß sie die Einspeisleitung (16) für Methanzusatz, mit der die Einspeisleitung (21) für aktives Mittel, halogenierte Verbindung, Wasserstoffsäure oder halogeniertes Derivat, und die Wiedergewinnungsleitung (12) für methanhaltiges Gas verbunden sind, dann die Einspeisleitung (15) für Stickstoffperoxidzusatz, mit welcher die Wiedergewinnungsleitung (14) für $NO_2$-reiches Gas verbunden ist, in welche die Erhitzer ($J_2$) und ($J_1$) zwischengeschaltet sind, einen Mischer für Fließmittel (1) und (2), die von den nicht gezeigten Erhitzern in der Nähe des Nitrierungsreaktors vom Röhrenfasertyp (A) stammen, die Kühler ($B_1$) und ($B_2$) für die Ausläufe (4), eine Trenneinrichtung (C), gegebenenfalls einen Wäscher (D), die Kolonne (E) zur Oxidation niederer Stickstoffoxide, die Kolonne zur Absorption von Stickstoffperoxid (F), einen Kompressor (H) für gasförmigen Ausfluß, der durch die Wiedergewinnungsleitungen (11) und (12) nach katalytischer Oxidation in der Oxidiereinrichtung (I) zu dem Nitrierungsreaktor zurückkehrt, und die Kolonne (G) für die Desorption von Stickstoffperoxid durch eine Gasfraktion, die die katalytische Oxidiereinrichtung (I) verläßt, aufweist.

# FIG.1

FIG.2